# EUROPEAN PATENT APPLICATION

(11) **EP 4 424 390 A1**
(43) Date of publication of application: **04.09.2024**
(21) Application number: 22887773.4
(22) Date of filing: 24.10.2022
(51) Int. Cl.: A63B 23/16, B25J 9/00, B25J 15/00

(54) **EXOSKELETON SYSTEM FOR ORTHOSES AND PROSTHESES OF HAND PHALANGES**

(30) Priority: 29.10.2021 MX 2021013325
(71) Applicant: Izaguirre Pérez, Paola Aralid, Col. Jardines Vallarta Jalisco, 4957 (MX)
(72) Inventor: Izaguirre Pérez, Paola Aralid, Col. Jardines Vallarta Jalisco, 4957 (MX)
(74) Representative: De Clercq & Partners
(86) International application number: PCT/MX2022/050102
(87) International publication number: WO 2023/075587

(57) **Abstract**

Various aspects of the present invention pertain to an exoskeleton system for orthoses and prostheses for phalanges, comprising a set of orthoprosthetic phalanges, a traction mechanism, and a coupling assembly, which operates solely mechanically based on the metacarpophalangeal joint movement of the patient, and therefore, the exoskeleton system of the present invention does not require electromechanical attachments, control, or derivatives to carry out system movement.

The present invention has been designed for use in patients who have had the distal and middle phalanges amputated from any of the thumb, index, middle, ring, and/or little fingers, or who, in any of the aforementioned fingers, present injuries, atrophy, paralysis, or any motor limitation requiring the assistance of the exoskeleton system of the present invention; and thus, still retain the proximal phalanx in any of the previously mentioned fingers.

The present invention can be employed in recent and mature amputations, as well as in injuries, atrophies, paralysis, and/or any motor limitation present in any of the patient's fingers, with the aim of providing the patient with these middle and distal phalanges again, and primarily to restore functionality related to both portions of the fingers, that is, extension and flexion thereof, where advantageously, each exoskeleton system of the present invention can operate independently, i.e., without needing to interact between different exoskeleton systems when the patient has multiple amputated fingers or if only one is used.

## Description

### Field of the invention

The present invention relates generally to an exoskeleton system comprising a series of aesthetic orthoprosthetic elements with a traction mechanism for use in orthoses and prostheses, in patients who have suffered amputations, particularly of the distal and middle phalanges. In this regard, the system of the present invention can be used in recent or mature amputations in any of the patient's thumb, index, middle, ring, and pinky fingers, independently of each other, provided that the proximal phalanx is retained.

The exoskeleton system of the present invention can be positioned/removed by the patient themselves in a simple, comfortable, and practical manner, adapting the exoskeleton system to the middle, proximal, or metacarpophalangeal phalanges of a patient's finger, depending on the level of amputation thereof; the exoskeleton system can be employed, particularly, in fingers with amputations of the distal and/or middle phalanges or in patients whose fingers exhibit any injury, for example, atrophy, paralysis, or deformity. Specifically, the system of the present invention can switch between an open or extended position and a closed or flexed position relying solely on the natural movement of the patient's hand, i.e., through the movement of the interphalangeal and/or metacarpophalangeal joint, tendons, and muscles associated with the movement of the corresponding finger, in conjunction with the traction mechanism of the exoskeleton system, that is, without an additional electromechanical or power element..

### Background of the invention

The implementation of exoskeleton devices or systems for use as orthoses and/or prostheses is increasingly widespread today; these devices allow for the replacement of different human body parts that have been amputated or removed, or in some cases, for limbs with injuries, atrophy, paralysis, or general motor limitations, with the aim of restoring the function associated with said limb.

For patients with amputated phalanges, known exoskeleton devices or systems for orthoses and/or prostheses within the prior art include cosmetic prostheses, whose function is limited exclusively to aesthetic purposes, therefore, they do not allow or are capable of restoring the function associated with the replaced body part; on the other hand, electromechanical prostheses configured to fit or be incorporated into the wrist and/or dorsal part of the patient's hand have been found within the prior art, as well as prosthetic modules that replace an amputated phalanx and, through a series of bars (bar mechanism), servomotors, control modules, and power sources, devices within the prior art allow for the extension or flexion of the aforementioned prosthetic modules; this is so that a patient who has been amputated of any of their fingers can regain the associated function.

This represents, firstly, high costs from planning and design to final production or manufacturing, as it is necessary to consult with a specialist in the manufacturing of biomechanical devices who has appropriate manufacturing means, so an average patient could encounter significant difficulties in acquiring and/or covering said device or system, additionally, it is known that said electromechanical systems can only be adapted when the patient has been fully amputated of the finger, that is, they do not retain any phalanx, so that the system can replace and carry out the entire function associated with the finger, with this, it is inconvenient or even impossible to adapt said prior art electromechanical systems to patients who still retain a phalanx, for example, middle and/or proximal.

Additionally, exoskeleton devices or systems within the prior art can be of large dimensions and weight, mainly due to the bar-type mechanisms incorporated on their exterior, thus complicating or limiting portability and practicality, as well as their aesthetics, features that are inconvenient for the daily life of patients, as well as for their adaptation process.

The above represents a relevant technical problem for patients who require a prosthesis or orthosis derived from one or more phalanx amputations, for whom it would not be feasible to obtain a system with specialized elements or components and complicating or limiting the patient's adaptation process due to the size of the device.

While some exoskeleton devices and/or systems for phalanges within the prior art include, as previously mentioned, bar mechanisms that move prosthetic phalanges to change or displace these from an extended to a flexed configuration and vice versa, it is clear that they employ control and power elements (microcontrollers, motors, power sources, readable means, etc.) to complete said stages.

Such is the case of the exoskeleton device described in Chinese patent application CN110695971 A published on January 17, 2020, which describes an electromechanical arm-assisting exoskeleton, comprising a mechanical structural component and a power component. The mechanical structural component has a wrist glove or sleeve, a plastic cover resembling a palm profile, and a set of articulated plastic fingers complementing said palm, and a linking structure between said elements. The power component consists of a directional motor and a pressure sensor electrically connected to the single-chip microcomputer control system; said directional motor is installed on the back of the plastic cover resembling a palm and is connected with the linking structure; on the other hand, the pressure sensor is installed in the fingertip portion.

On the other hand, Chinese patent CN109223442 B published on February 18, 2020, discloses an electromechanical exoskeleton manipulator for rehabilitation with seven degrees of freedom, which comprises a four-finger bending mechanism, a unit with a finger stretching mechanism, a hand support unit, and a unit with a thumb bending mechanism. The four-finger bending, finger stretching, and thumb bending mechanism units are respectively connected to the back of the hand support unit. This device is configured to drive a PIP joint and an MP joint so that each finger rotates, flexes, or stretches independently.

Meanwhile, the article titled "Investigation on a New Hand Exoskeleton Structure for Rehabilitation" published in 2017 in the Fourth International Conference on Information, Science, and Control Engineering (ICISCE), discloses a portable manual exoskeleton device that can adapt to the patient's hand through a base connected to a set of rigid structures and on the other hand, to an electromechanical mechanism corresponding to the proximal, middle, and distal phalanges that adjust over the patient's finger. The previously referred mechanism corresponding to the phalanges allows for the extension or flexion of the patient's phalanges through the motor and a set of coupling tendons (cross bands), which allow for mechanical interaction between the distal and middle phalanx.

Another document found within the prior art is Chinese patent CN109223442, published on January 18, 2019, which discloses an exoskeleton system incorporating a support to position the referenced system on the dorsal part of the patient's hand, and on the other hand, the CN109223442 patent system comprises a set of actuators (electromechanical units) connected to a unit of connectors or bar-type mechanisms configured to move prosthetic phalanges, particularly allowing extension and flexion movements, respectively.

From the teachings of the prior art, it is clear that, to date, exoskeleton devices or systems for phalange prostheses can perform the extension and/or flexion movement of a patient's phalanges, whether the patient's own phalanges (in cases where they are retained) or prosthetic phalanges, solely through electronically assisted mechanisms or electromechanical power units, implying, as previously mentioned, the use or interaction with specialized components such as controllers and/or microcontrollers, motors, servomotors, actuators, power sources, etc.; therefore, there is a need to provide an exoskeleton device or system for phalange orthoses or prostheses that can operate without the need for electronic components or electronically assisted components, i.e., solely mechanical, in order to significantly reduce planning, design, and manufacturing costs, obtaining a more economical, simple, and accessible device for the patient compared to current systems. Additionally, there is a need to provide an exoskeleton device or system that allows for the recovery of finger functionality, as well as the entire hand of the patient, even when the patient still retains some phalanges such as middle and/or proximal, and eliminating the need for a system that can only be adapted if the patient lacks any phalanx, as cited in this application, which constitute some examples of devices or systems within the prior art.

Furthermore, as mentioned previously in this document, the movement of the patient's phalanges (own or prosthetic) is directly linked or dependent on an external mechanism (generally bar-type mechanisms) connected to said phalanges and, on the other hand, to a motor, servomotor, actuator, and/or any other known electromechanical driver arranged on a base, glove, support, or structure to attach the system to the dorsal part of the patient's hand. However, it is clear that to accommodate or arrange such components, the disclosed system must be of large dimensions, albeit suitable for denoting them as surplus, resulting in an impractical device or system, both for attaching and accommodating it on the user's hand and respective phalanges, as well as during its interaction with the user, i.e., during use, further disfavoring aesthetics and mainly portability. Therefore, there is a need for a functional and practical exoskeleton device or system for phalange orthoses and prostheses of the patient's hand, which can be ergonomically, practically, comfortably, and simply attached and adapted to the patient's remaining stump, i.e., that can be incorporated and removed from the middle and/or proximal phalanges and/or metacarpophalangeal joint of the patient's hand, and which, in general, has smaller dimensions, less weight, and additionally features an ergonomic, aesthetic, and practical design that integrally facilitates the patient's adaptation to said prosthetic system.

### Summary of the Invention

The present invention aims to provide an exoskeleton system for phalange orthoses and/or prostheses, configured to provide extension and flexion movement independently in any of the thumb, index, middle, ring, and little fingers, for patients with recent and/or mature amputations of the distal and middle phalanges, while preserving the proximal phalanx. This system is capable of operation without any electronic and/or power components, solely through a mechanical means relying on the natural movement of joints, tendons, and associated muscles in the flexion-extension of the respective phalanx, aiming to restore movement and functionality associated with the patient's hand finger where the exoskeleton system will be used, particularly to provide flexion-extension movement, as well as pinch movement and gripping force.

By "system," within the context of the present invention, it should be understood as a set of components or elements configured to interact with each other, which together enable the stated objective, although each of the elements of this system can operate independently, their relationship with at least some of the other elements forms a unified whole.

Another objective of the present invention is to provide an exoskeleton system with an ergonomic design that allows for simple, easy, comfortable, practical, and safe coupling/decoupling from the patient's hand and respective phalanx, protecting the residual stump of the patient's hand finger where the proposed system will be used. Additionally, due to its design and arrangement, the system is capable of reducing the adaptation process time and offering more practical and comfortable use for the patient.

Given the widespread use of various devices with touch or capacitive screens, another objective of the present invention is to provide an exoskeleton system with an interaction means integrated therein, allowing the patient to use and/or interact directly with touch and/or capacitive screens using the proposed exoskeleton system.

### Brief description of the drawings

Figure 1 is a top front perspective view of the exoskeleton system according to the present invention.
Figure 2 corresponds to an exploded top front perspective view of the exoskeleton system according to the present invention.
Figure 2a is a detailed front perspective view of the displaceable ring of the exoskeleton system according to the present invention.
Figure 3 is a side view of the exoskeleton system according to the present invention, excluding the multiperforated connector.
Figure 4 is a bottom front perspective view of the exoskeleton system according to Figure 3.
Figure 4a is a detailed view of the exoskeleton system according to Figure 4.
Figure 5 is an exploded top front perspective view of the exoskeleton system of the present invention, showing the prosthetic phalanges, coupling ring, and the first traction assembly.
Figure 6a is a top front perspective view of the exoskeleton system of the present invention, in an extended configuration of the prosthetic phalanges relative to the coupling ring.
Figure 6b is a top front perspective view of the exoskeleton system of the present invention, in a flexed configuration of the prosthetic phalanges relative to the coupling ring.

### Detailed description of the invention

Some aspects of the present invention will now be described in more detail with reference to the accompanying drawings showing various embodiments and advantages of the present invention.

For one skilled in the art, it will be evident that various embodiments of the invention can be expressed in many different forms and should not be interpreted as limited to the embodiments described herein; rather, these exemplary embodiments are provided to make the invention clear and complete and to fully convey the scope of the invention to those skilled in the art. For example, unless otherwise indicated, something described as first, second, or similar should not be interpreted as a particular order. As used in the description and in the accompanying claims, singular forms "a," "an," and "the" include plural referents unless the context clearly indicates otherwise.

Different aspects of the present invention relate to an exoskeleton system for orthoses and prostheses for phalanges comprising a set of prosthetic phalanges, a traction mechanism, and a coupling assembly, which operates solely mechanically based on the movement of the patient's metacarpophalangeal joint; therefore, the exoskeleton system of the present invention does not require electromechanical attachments, controls, or derivatives to perform the system's movement.

The present invention has been designed for use in patients who have had their distal and middle phalanges amputated, in any of the index, middle, ring, and/or little fingers, and therefore still retain the proximal phalanx in any of the aforementioned fingers.

The present invention can be used in recent as well as mature amputations, to provide the patient again with these middle and distal phalanges and, primarily, to restore functionality related to both portions of the fingers, i.e., extension and flexion thereof, wherein advantageously, each exoskeleton system of the present invention can operate independently, i.e., without the need to interact between different exoskeleton systems when the patient has multiple amputated fingers or if only one is employed.

The exoskeleton system of the present invention is configured to be coupled to the proximal phalanx or remaining stump of the amputation of the distal and middle phalanges.

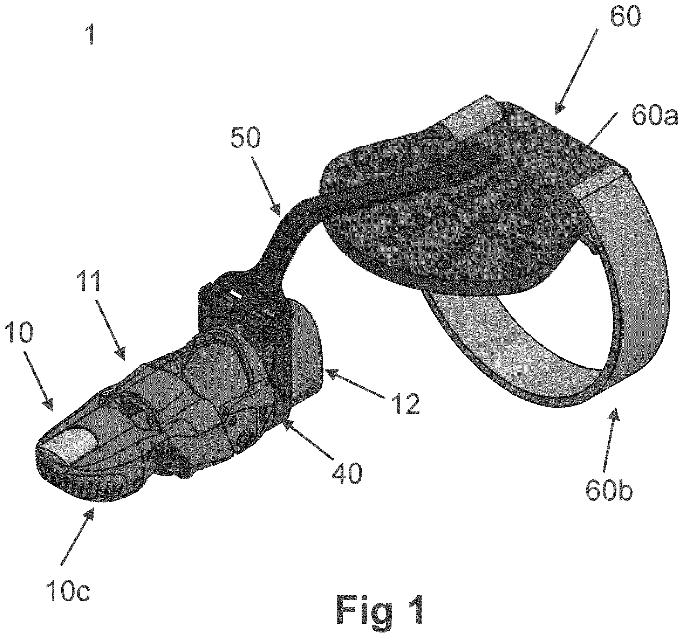

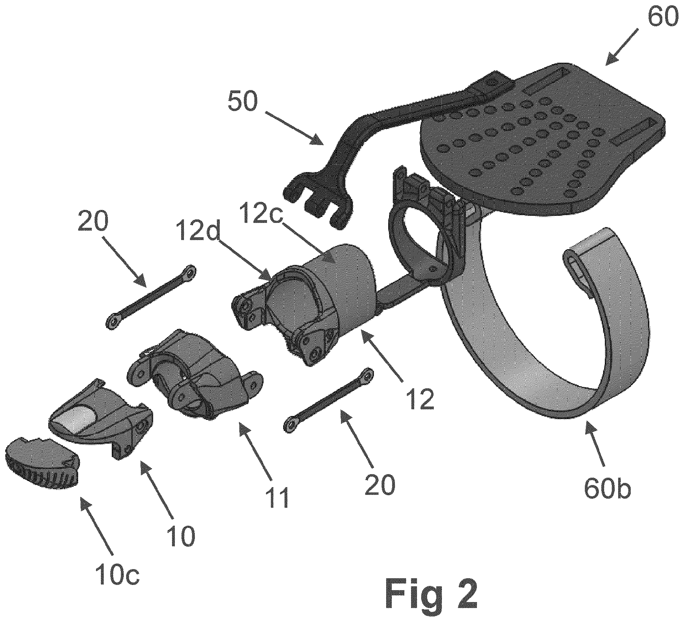

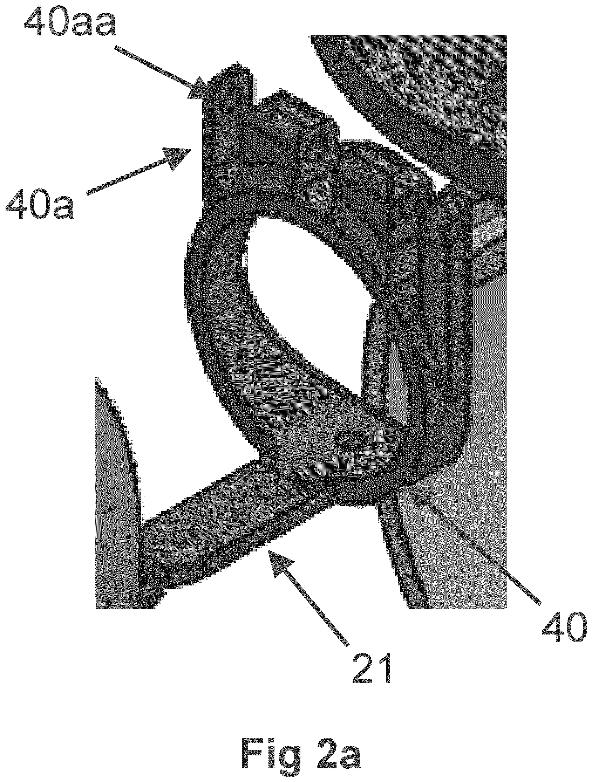

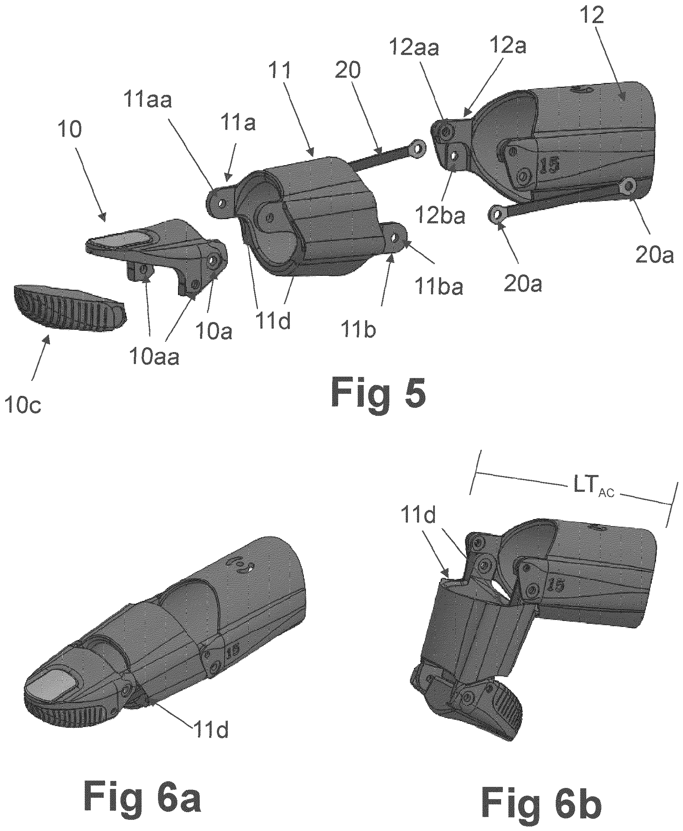

As can be seen in Figures 1 and 2, in a preferred embodiment, the exoskeleton system (1) of the present invention comprises three main parts:
a) set of prosthetic phalanges (10, 11), which comprises a distal prosthetic phalanx (10) and a middle prosthetic phalanx (11) designed to simulate the anatomy, aesthetics, and functionality of distal and middle phalanges, respectively, of a human being as observed in Figures 1 and 5.

In this regard, the distal prosthetic phalanx (10) is pivotally coupled with the middle prosthetic phalanx (11) via appropriate fastening means, as will be described later in this application.

The distal prosthetic phalanx (10) includes, on each side, a coupling receiving tab (10a) with a bore (10aa) that, as will be described later, allows for adapting the corresponding bore of the middle prosthetic phalanx (11).

Additionally, the distal prosthetic phalanx (10) includes a bore (10ab) on each side, which, as will be described later, allows for the coupling of a first internal traction assembly (20).

Furthermore, the distal prosthetic phalanx (10) includes a traction surface (10c) disposed on the front bottom as observed in Figures 1 and 5; the traction surface (10c), possibly made of a flexible and non-slip material, is capable of elastically deforming when it comes into contact with a specific object so that the patient can grasp, hold, or grip irregular objects more precisely and firmly when using the exoskeleton system of the present invention.

The traction surface (10c) is designed to resemble or analogously to the fingertip area of a human finger, allowing for grasping objects when a force is applied thereto.

By "a flexible and non-slip material," it should be understood as any suitable material that can elastically deform, i.e., capable of returning to its original shape when the interaction of said component with the object in question concludes, and moreover, said material allows for firmly and comfortably holding any object regardless of its shape; such materials can be and are not limited to silicones such as conductive silicone, silicone with a mixture of carbon black, among others, rubbers, EVA foam, rubber, silicone, polyethylene, texovinyl, nitrile, neoprene, latex, PVC, and/or any combination thereof.

In an additional embodiment, the traction surface (10c) of the distal prosthetic phalanx (10), possibly made of any of the aforementioned materials, to obtain the referred characteristics, can also be made with a material compatible with touch devices, touch-sensitive or capacitive devices such as smartphones, tablets, smartwatches, touchscreens, among others.

By "a material compatible with touch devices," it should be understood as any suitable material that, upon contact with the interface, typically a touchscreen or touch device, said touch device can recognize gestures, movements, and generally any interaction performed by the user of the system of the present invention; such materials can be any from the selected group comprising silicones such as conductive silicone, silicone with a mixture of carbon black, among others, rubbers, EVA foam, rubber, silicone, polyethylene, texovinyl, nitrile, neoprene, latex, nylon, PVC, and/or any combination thereof.

Regarding the middle prosthetic phalanx (11), said component includes, on each of its sides, front coupling tabs (11a) with a bore (11aa), configured to concentrically match the bores (10aa) of the coupling tabs (10a) of the distal prosthetic phalanx (10), such that when said bores (10aa, 11aa) of both front coupling tabs (10a and 10b) concentrically match, they house the necessary fastening means to allow pivoting connection between said components.

Furthermore, the middle prosthetic phalanx (11) includes, on each of its sides, rear coupling tabs (11b), with a bore (11ba), which similarly to the front coupling tabs (11a), said rear tabs (11b) allow for the coupling of the middle prosthetic phalanx (11) with a coupling ring (12) which will be described in greater detail later on.

In this regard, when the bores (11ba) of the rear coupling tabs (11b) of the middle prosthetic phalanx (11) concentrically match with bores (12aa) arranged on a coupling tab (12a) of the coupling ring (12), the necessary fastening means are housed to allow pivotal connection between these components.

By "fastening means," it should be understood as any element that serves to appropriately fix and/or secure the different components of the present invention; thus, for the coupling between the distal and middle prosthetic phalanges (10, 11) and the middle prosthetic phalanx (11) with the coupling ring (12), suitable fastening means can include but are not limited to bolts, rivets, pins, screw-nut systems, among others, that allow only rotary movement of one component relative to another as previously described.

The middle prosthetic phalanx (11) symmetrically comprises, relative to the center of the exoskeleton system (1), a pair of lower coupling tabs (11c) beneath the body of said phalanx (11), spaced apart and each with a bore (11cb), which, as will be described later, allows for the coupling of a second internal traction assembly (21).

Additionally, as seen in Figures 6a and 6b, the middle prosthetic phalanx (11) includes, on each side internally, grooves (11d) functioning as a sleeve, enclosure, or guide for the first internal traction assembly (20), allowing them to move within it, as will be described in greater detail later on.

In the embodiment depicted in this application, the length of the prosthetic phalanges (10, 11) ranges from 5mm to 150 mm, wherein the middle prosthetic phalanx (11) may be longer than the distal prosthetic phalanx (10) to maintain a similar proportion to human phalanges.

b) A coupling ring (12), comprising two relevant sections or portions:
i) an elongated hollow geometric body (12c) configured firstly to position and secure inside it the proximal phalanx of the patient, thus allowing the patient to place the exoskeleton system on their amputated finger, and additionally, the elongated hollow geometric body (12c) acts as an axis for the movement or stroke of an external traction assembly (40,50), which will be described later in this application.

By "elongated hollow geometric body," any volumetric body with a certain height and a hollow interior allowing the correct passage of the patient's proximal phalanx into the coupling ring (12) of the system of the present invention should be understood. This also facilitates the positioning and securement of said ring (12) to the patient's proximal phalanx in a comfortable, firm, and secure manner; such bodies can be prisms, such as hollow circular and/or elliptical cylinders, hollow conical cylinders, hollow polygonal prisms, combinations thereof, among others.

The coupling ring (12) can house and secure any size and dimension of a patient's proximal phalanx, such that the exoskeleton system of the invention can be used on any of the index, middle, ring, and/or little fingers independently.

By "independently," it should be understood that the present invention can be placed on any of the aforementioned fingers and can operate isolated or separately from each other. For example, if the patient does not have the middle and distal phalanges of the index and ring fingers, it is possible to adapt an exoskeleton system as described herein to each finger, and the feature of operating independently allows each to function separately, without one depending on or interacting with the system placed on the other finger.

In this sense, the coupling ring (12) can have an inner diameter ranging from 10 mm to 50 mm and a total length (LTAC) from 5 mm to 60 mm.

Additionally, the distal (10) and middle (11) prosthetic phalanges referred to herein can have proportional, similar, or identical dimensions to the diameter of the coupling ring (12), so that these prosthetic phalanges (10, 11) are uniform or demonstrate continuity relative to the coupling ring (12), enhancing the shape and aesthetics of the exoskeleton system of the invention.

Furthermore, the coupling ring (12), as well as the prosthetic phalanges (10, 11) can have a thickness ranging from 2 mm to 20 mm and can be manufactured by any appropriate production method, using materials selected from the group comprising polyvinyl chloride (PVC), low-density polyethylene (LDPE), polypropylene (PP), polystyrene (PS), flexible polyethylene, urethane, nylon, and/or any combination thereof, as well as metals such as steel, titanium, aluminum, alloys, mixtures thereof, copolymers, and/or any suitable ductile material.

By "any appropriate production method," it should be understood as any method, procedure, or manufacturing process known today that allows for forming the components of the present invention as described, such as plastic injection molding, thermoforming, rotomolding, blow molding, 3D printing, molding, or combinations thereof, among others.

ii) a protruding portion (12d) which, on one side, allows coupling with the middle prosthetic phalanx (11) through coupling tabs (12a), and furthermore, said protruding portion (12d) is configured to delimit a maximum stroke of the external traction assembly (40,50), which will be described later in this document.

The front coupling tabs (12a) of the coupling ring (12) are arranged on both sides of the coupling ring (12) as can be appreciated in Figures 1, 2, and 5, wherein said tabs (12a) each comprise: a first bore (12aa), which, when arranged concentrically with the bore (11ba) of the rear coupling tab (11b) of the middle prosthetic phalanx (11), allows pivotal connection between both components by providing an appropriate fastening means within it, as previously described, and also comprises a second bore (12ab), which, as will be described later, will be joined with the first internal traction assembly (20).

The coupling ring (12) further comprises a groove (12dd) arranged on the bottom part of the protruding portion (12d) functioning as a sleeve, envelope, or guide for the second internal traction assembly (21), allowing it to move within it, forcing or limiting the displacement of said assembly (21) to be normal to the groove, and additionally, said groove (12dd) is configured to delimit a minimum stroke of the external traction assembly (40,50), as will be described later in this document.

The protruding portion (12d) may have a diameter larger than the diameter of the hollow elongated geometric body (12c), for example, the diameter of the protruding portion (12d) may be from 2 mm to 10 mm larger than the diameter of the hollow elongated geometric body (12c).

c) traction mechanism, which is responsible for modifying the state of the exoskeleton system of the present invention, i.e., from a flexed to an extended configuration and vice versa based on the movement of the patient's metacarpophalangeal joint. The traction mechanism is divided into two assemblies:
i) an internal traction assembly (20,21), which is considered "hidden" as it is disposed inside the middle and distal prosthetic phalanges (11, 10), as will be described later; the internal traction assembly (20,21) further comprises a first internal traction assembly (20) and a second internal traction assembly (21).

The first internal traction assembly (20) is composed of two rectangular bars with a bore (20a) at each end thereof; said bars (20) are arranged on both sides of the exoskeleton system (1) of the present invention.

As can be seen in Figures 2 and 5, the first internal traction assembly (20) connects or allows the connection between the bottom of the distal prosthetic phalanx (10) and the top of the protruding portion (12d) of the coupling ring (12) when the respective bores of the bar (20a) concentrically match the bores (10ab) of the distal prosthetic phalanx (10) and the bores (12ab) of the protruding portion (12d) of the coupling ring (12).

On the other hand, the second internal traction assembly (21) is a single rectangular bar with a bore (21b) at each end thereof; said bar (21) is centrally disposed at the bottom of the exoskeleton system (1).

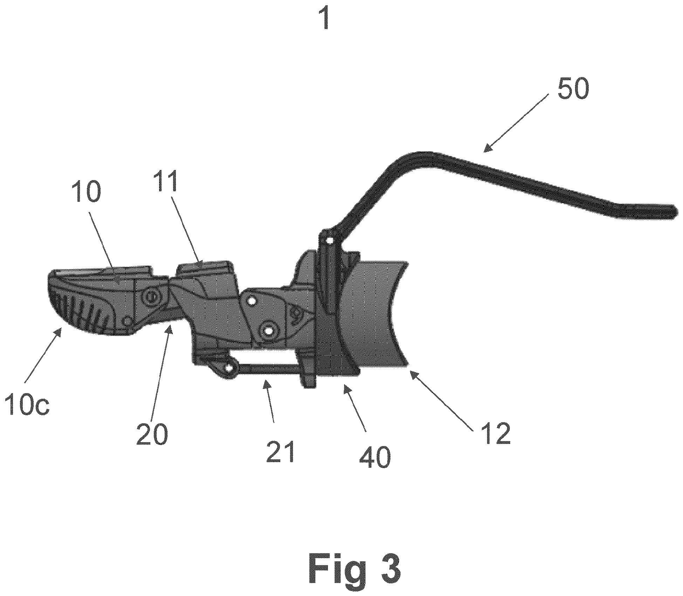

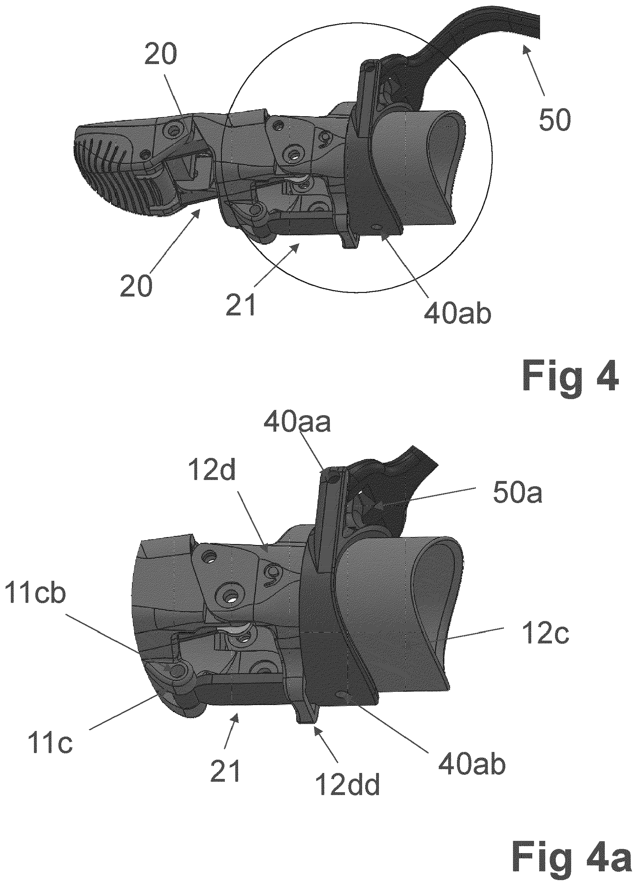

As observed in Figures 2 to 4a, the second internal traction assembly (21) connects or allows the connection between the bottom of the middle prosthetic phalanx (11) and the bottom of a movable ring (40) when the respective bores of the bar (21b) concentrically match holes (40a) of the movable ring (40) and the bores (11cb) of the lower coupling tabs (1 1c) of the middle prosthetic phalanx (11).

It should be noted that for both the first and second internal traction assemblies (20,21), they are coupled to the respective prosthetic phalanges (10, 11) as previously described, by means of an appropriate fastening means.

Furthermore, the first and second internal traction assemblies (20,21) can have dimensions ranging from 2 cm to 10 cm and can be manufactured from any type of metallic material, such as steel, aluminum, titanium, alloys, combinations thereof, and/or any other material resistant to bending and cutting forces resulting from the extension/flexion process carried out by the exoskeleton system of the present invention.

ii) an external traction assembly (40, 50, 60) which in turn, is divided into three components: a movable ring (40), an extender or lever (50), and a multi-perforated connector (60).

The movable ring (40) is a short hollow geometric body, configured firstly to horizontally slide over the hollow elongated geometric body (12c) of the coupling ring (12), secondly to be solidly connected to the bar of the second internal traction assembly (21), and additionally, said movable ring (40) can pivotally connect to the extender or lever (50).

Consequently, the movable ring (40) comprises a bore (40ab) arranged on its lower portion configured to, as mentioned previously, concentrically match with the end bore (21a) of the second internal traction assembly (21) and subsequently accommodate an appropriate fastening means that securely joins them.

On the other hand, the movable ring (40) comprises on its upper part and symmetrically with respect to the center of the exoskeleton system (1), spaced coupling tabs (40a), each with a bore (40aa), configured to concentrically match a bore (50a) of the extender or lever (50) and subsequently accommodate an appropriate fastening means that pivotally joins them.

The movable ring (40) has the same shape as the hollow elongated geometric body (12c) of the coupling ring (12), so that said movable ring (40) can freely move vertically along the length of the referenced body (12c) of the coupling ring (12).

"Freely movable" shall be understood as the continuous movement of the displaceable ring (40) along the referenced body (12c), requiring both geometries to match to prevent the displaceable ring (40) from encountering any protrusion due to differing geometries between the two bodies.

The displaceable ring (40) may have an identical or slightly smaller diameter than the protruding portion (12d) of the coupling ring (12) and additionally, the displaceable ring (40) has a diameter larger than the diameter of the hollow elongated geometric body (12c) of the coupling ring (12), enabling the displaceable ring (40) to move freely vertically along the length of the hollow elongated geometric body (12c) of the coupling ring (12).

The extender or lever (50) is configured to first connect with the displaceable ring (40) as previously described, and on the other hand, to connect with the multi-perforated connector (60).

In this regard, the extender or lever (50) has been designed with a curved shape to promote resistance, hardness of the element, and efficient and adequate transfer of movement derived from the patient's hand to the displaceable ring (40), allowing switching between flexion or extension configurations respectively. However, the shape or expanded design may vary substantially, maintaining the previously mentioned functions and characteristics, and therefore, remaining within the scope of the present invention.

The extender or lever (50) includes at each of its ends, a borehole (50a), configured to respectively and concentrically match the borehole (40aa) of the coupling tab (40a) of the displaceable ring (40), subsequently housing an appropriate fastening means that pivotally joins as previously described, and on the other end, can align with one of the boreholes (60a) of the multi-perforated connector (60) as detailed later in this application.

With reference to the multi-perforated connector (60), this component is configured to adapt to the dorsal part of the patient's hand using an appropriate fastening assembly (60b).

By "appropriate fastening assembly," any coupling or fastening means allowing the connector (60) to be attached or secured to the dorsal part of the patient's hand or the dorsal part of the hand along with the patient's wrist, such that there is no relative movement between the connector (60) and the patient's hand; such as, for example, strap/buckle systems, clamp systems, with mechanical adjustment means like butterfly screws, ropes or flexing elements, commercially available adjustment systems, such as the Boa Fit ^{®} system marketed by BOA^{®}, any combination thereof, among others.

As seen in Figures 1 and 2, the multi-perforated connector (60) can be a flat plate with a fan-like shape with numerous perforations (60a) arranged radially relative to the upper curvature of the connector (60); additionally, the multi-perforated connector (60) can adopt other geometries, regular or irregular, with perforations (60a) distributed in different patterns, orders, or arrangements not limited to the embodiment represented in this document and/or accompanying Figures; this allows the connector (60) to adapt appropriately to the dorsal part and/or wrist of the patient, enabling the traction function of the exoskeleton system (1) as described in this application.

The perforations (60a) are configured to receive, in any of them, the borehole (50a) of the extender or lever (50) end as previously described, so that the patient can adapt the extender or lever (50) to the perforation (60a) of the multi-perforated connector (60) that best accommodates the dimensions of their hand.

For example, a patient with a larger hand, or where the distance between their remaining stump (proximal phalanx) of the finger to which the exoskeleton system (1) is to be applied and the dorsal part of their hand is greater, the perforations (60a) will allow the patient to select the borehole that best fits; likewise, the perforations (60a) can accommodate the extender or lever (50) regardless of the finger where the exoskeleton system (1) is applied, i.e., through different rows of perforations in the connector (60).

On the other hand, the displaceable ring (40), the extender or lever (50), and the multi-perforated connector (60) can be made of any material selected from the group comprising polyvinyl chloride (PVC), low-density polyethylene (LDPE), polypropylene (PP), polystyrene (PS), flexible polyethylene, nylon, urethane, and/or any mix thereof, where like the prosthetic phalanges (10, 11) and the coupling ring (12), or can be made of any material selected from the group comprising steel, titanium, alloys, aluminum, combinations thereof, copolymers, and/or combinations with the materials previously referred to and/or any other material providing sufficient resistance to bending, compression, shear, among others.

The length of the extender or lever (50) can be from 5 cm to 30 cm measured horizontally (in projection), from each borehole (50a) arranged at both ends of the referenced component.

Regarding the multi-perforated connector (60), this component can have from 1 to n number of perforations, with a diameter from 2 to 10 mm and a spacing from 1 mm to 10 mm in any appropriate arrangement, i.e., it can be a radial arrangement, longitudinal, with a predefined pattern such as rectangular, circular, rectangular, polygonal, among others.

Likewise, the shape or geometry, as well as the overall dimensions of the connector (60) are not limited to those illustrated in the accompanying figures, an expert in the field will understand that any dimension, shape, or geometry can be provided to said component as long as it allows practical, comfortable, and efficient coupling to the dorsal part of the hand and/or wrist of the patient and, on the other hand, allows housing enough perforations to facilitate the adaptation of the exoskeleton system (1) to the dimensions and shapes of the patient's hand and proximal phalanx; in particular, the multi-perforated connector (60) can adopt a regular shape like a rectangle, circle, square, polygon or an irregular shape like a fan, half-circle, trapezoid, or combinations thereof, or in the shape of a glove, and can measure from 5 cm to 15 cm with reference to the shape adopted by said connector (60) circumscribed in a circle with these measurements.

### EXAMPLE OF OPERATION

The following is an example of operation of the exoskeleton system of the present invention, which should not be interpreted as limiting, as it is provided to complement and facilitate understanding of the claimed invention.

Once the exoskeleton system of the present invention has been fully assembled, i.e., by connecting as described in this application, the prosthetic phalanges (10, 11) together, the middle prosthetic phalanx (11) with the coupling ring (12), the internal (20, 21) and external (40, 50, 60) traction assemblies together, and finally, the patient positions the multi-perforated connector (60) on the dorsal part of their palm and their proximal phalanx (remaining stump of the patient) within the coupling ring (12) of the exoskeleton system (1), the present invention can switch between two states or configurations:
i) Extension configuration, when the distal and middle prosthetic phalanges (10, 11) are fully aligned with the patient's proximal phalanx, i.e., they are fully extended or straight, and therefore, there is no angular difference between these prosthetic phalanges (10, 11) and the patient's proximal phalanx.
ii) Flexion configuration, when the distal and middle prosthetic phalanges (10, 11) are in any relative position with respect to the patient's proximal phalanx, i.e., when there is a non-zero angle between the position of the prosthetic phalanges (10, 11) and the patient's proximal phalanx.

The switching between the aforementioned states or configurations is carried out by the movement of the respective metacarpophalangeal joint of the finger interacting with the exoskeleton system (1) of the present invention.

When the patient desires the exoskeleton system (1) to remain in an extended configuration, the patient's proximal phalanx is aligned (or maintained at zero degrees) relative to the corresponding metacarpal bone; alternatively, when the patient desires the exoskeleton system (1) to switch to a flexion configuration, the patient's proximal phalanx is at a certain angle (non-zero degrees) relative to the corresponding metacarpal bone.

Thus, if the distance between any point of the patient's proximal phalanx and the selected perforation (60a) where the extender or lever (50) is attached to the multi-perforated connector (60) is measured, this distance is smaller in the extended configuration than in the flexion configuration. Due to this difference in distances or displacements derived from the movement of the patient's metacarpophalangeal joint, it allows:

When the exoskeleton system (1) of the present invention is in the extended configuration, the extender or lever (50) drives and pushes the displaceable ring (40), moving it along the hollow elongated geometric body (12c) until a minimum stroke (when the displaceable ring (40) comes into contact with the protruding portion (12d) of the coupling ring (12)), which integrally displaces both bars of the internal traction assemblies (20, 21), functioning similarly to a parallel bar mechanism, forcing the prosthetic phalanges (10, 11) to align with the coupling ring (12); and

When the exoskeleton system (1) of the present invention transitions from the extended configuration to the flexion configuration, the extender or lever (50) guides, pulls, or draws the displaceable ring (40), moving it along the hollow elongated geometric body (12c) until a maximum stroke (when the displaceable ring (40) moves away from the protruding portion (12d) of the coupling ring (12), proximally towards the patient's hand), which integrally displaces both bars of the internal traction assemblies (20, 21), functioning similarly to a parallel bar mechanism, forcing the prosthetic phalanges (10, 11) to rotate, generating an angle from 0° up to approximately 90°, from 0° up to 95°, from 0° up to 85°, measured from the middle prosthetic phalanx (11) relative to the coupling ring (12), with the coupling ring (12).

Based on the above, when the patient moves their proximal phalanx to switch between any of the configurations described above, advantageously, the exoskeleton system (1) of the present invention can remain in the respective configuration, even when there is an external force opposing or attempting to displace the prosthetic phalanges (10, 11), until the patient rotates their proximal phalanx again.

For example, when the exoskeleton system (1) is in a flexion configuration, the prosthetic phalanges (10, 11) will maintain their relative position to the coupling ring (12) as described previously (i.e., at a non-zero angle), even when there is a counterforce resulting when the patient attempts to grasp an object between the distal prosthetic phalanx (10) of an exoskeleton system (1) on an amputated finger and the patient's own distal phalanx or between two prosthetic phalanges (10, 11) of two independent exoskeleton systems (1) placed on different amputated fingers of the patient; consequently, the exoskeleton system (1) will not change configuration until the patient rotates their proximal phalanx. This applies similarly in the extension configuration.

In this regard, Figures 6a and 6b respectively illustrate both extension and flexion states or configurations that can be switched by the exoskeleton system (1) according to the present invention, through the mechanism and method described in this application.

Many modifications and other embodiments of the invention will come to the mind of one skilled in the art to which the invention pertains, having the benefit of the teachings presented in the foregoing descriptions and associated drawings. Therefore, it should be understood that the invention should not be limited to the specific and exemplary embodiments described, but that modifications and other embodiments are intended to be included within the scope of the appended claims. Although specific terms are employed herein, they are used only in a generic and descriptive sense and not for purposes of limitation. Additionally, it should be understood that the materials from which the various components of the invention described in this document can be made, the geometries, dimensions, arrangements, and other elements may vary without departing from the scope and spirit of the invention and therefore, the embodiments described should not be considered limiting.

Below is the list of elements with their respective identifiers.
1 exoskeleton system
10 distal prosthetic phalanx
10a distal coupling tab
10aa distal coupling tab borehole
10ab receiver borehole for first internal traction set (20) of distal prosthetic phalanx.
10c traction surface
11 medial prosthetic phalanx
11a frontal coupling tab (medial-proximal phalanx junction)
11aa frontal medial coupling tab borehole
11b posterior coupling tab (medial-proximal phalanx junction)
1 1ba medial coupling-anterior ring coupling borehole
11c lower coupling tabs of medial prosthetic phalanx
1 1cb receiver borehole for second internal traction set (21)
11d lower slot of medial prosthetic phalanx (guide for second traction set 21)
12 coupling ring
12a medial-prosthetic phalanx coupling tab.
12aa proximal coupling tab borehole of coupling ring
12ab proximal coupling borehole with distal-proximal traction system
12c elongated hollow geometric body.
12d protruding portion.
12dd lower slot of protruding portion (guide for second internal traction set)
20 first internal traction set.
20a borehole at each end of first internal traction set.
21 second internal traction set.
21a borehole at each end of second internal traction set.
40 displaceable ring
40a upper ring coupling tab (connect displaceable ring-extension)
40aa upper displaceable ring coupling tab borehole
40ab lower displaceable ring borehole (to connect second internal traction set)
50 extender or lever
50a borehole at each end of extender or lever (connect displaceable ring and multi-perforated connector)
60 multi-perforated connector
60a multi-perforated connector boreholes

## Claims

1. A mechanical exoskeleton system (1) for prostheses and orthoses capable of adapting to any of the thumb, index, middle, ring, and/or little fingers of a patient who has suffered amputations of distal and/or middle phalanges and/or has any injury, atrophy, paralysis, or motor limitation in any of the aforementioned fingers, wherein the exoskeleton system (1) can be independently adapted to each other, i.e., without requiring a first exoskeleton system (1) on one amputated or injured finger of the patient to interact with a second exoskeleton system (1) on another finger different from the first, and wherein furthermore, at least one and up to 5 exoskeleton systems (1) can be placed respectively on each of the patient's hand fingers simultaneously,
wherein the exoskeleton system can be used in patients with recent and mature amputations, as well as in patients with partial injuries involving a motor limitation, thus requiring assistance from the exoskeleton system (1), in any of the aforementioned patient fingers, in order to mainly restore functionality associated with the amputated middle and distal phalanges of the patient, the system comprises:
a set of orthopedic fingers, designed to simulate the anatomy, aesthetics, and functionality of the middle and distal phalanges, respectively, of a human being;
a coupling assembly, allowing the exoskeleton system (1) to be attached, secured, and adapted to the proximal phalanx (remaining stump) of the patient; and
a traction mechanism capable of transmitting the movement of the metacarpophalangeal joint of the patient's hand to the prosthetic phalange set, allowing switching between a flexion configuration and an extension configuration.

2. The exoskeleton system according to claim 1, wherein the prosthetic finger set includes a distal prosthetic phalanx (10) and a middle prosthetic phalanx (11) pivotally connected to each other through bores (10aa, 11aa) arranged in coupling tabs (10a, 11a).

3. The exoskeleton system according to claim 1 and 2, wherein the distal prosthetic phalanx (10) further comprises a traction surface (10c) disposed on the front bottom of said prosthetic phalanx (10),
wherein the traction surface (10c) is made of a flexible and non-slip material capable of elastically deforming when in contact with a specific object so that the patient can grip, hold, or grasp irregular objects more precisely and firmly,
wherein the traction surface (10c) is made of a material selected from the group consisting of conductive silicone, silicone with carbon black blend, rubbers, EVA foam, rubber, silicone, polyethylene, texovinyl, nitrile, neoprene, latex, nylon, PVC, and/or any combination thereof, and
wherein the traction surface (10c) is also made of a material compatible with touch or capacitive devices, such as smartphones, tablets, smartwatches, touch screens, among others, allowing the patient to use any of the aforementioned devices when using the exoskeleton system (1), particularly when interacting with touch-enabled devices using the traction surface (10c) of the distal prosthetic phalanx (10).
wherein the material compatible with touch or capacitive devices is any selected from the group consisting of conductive silicone, silicone with carbon black blend, rubbers, EVA foam, rubber, silicone, polyethylene, texovinyl, nitrile, neoprene, latex, nylon, PVC, and/or any combination thereof.

4. The exoskeleton system (1) according to claim 2, wherein the middle prosthetic phalanx (11) further comprises
posterior coupling tabs (11b) each with a bore (11ba) allowing the middle prosthetic phalanx (11) to be joined with a coupling ring (12),
a lower coupling tab (11c) with a bore (11cb) configured to align with a bore (21a) of an internal second traction assembly (21), and
an internal lower groove (11d) serving as a guide, sleeve, or enclosure for a first internal traction assembly (20), allowing movement of said first assembly (20) through the internal part of the middle prosthetic phalanx (11).

5. The exoskeleton system (1) according to claim 4, wherein the coupling ring (12) further comprises
a hollow elongated geometric body (12c) of any shape selected from the group consisting of prisms, such as hollow cylinders, hollow conical cylinders, elliptical cylinders, and
a protruding portion (12d),
wherein the coupling ring (12) is capable of connecting, on one side, to the middle prosthetic phalanx (11) through coupling tabs (11a, 12a) arranged on the protruding portion (12d), wherein said tabs (11a, 12a) comprise bores (11aa, 12aa) configured to concentrically align allowing the union between said components (11,12),
wherein furthermore, the hollow elongated geometric body (12c) is configured to contain, secure, and adapt to the patient's proximal phalanx, thus allowing the patient to wear the exoskeleton system (1) for use, and
wherein the protruding portion (12d) further comprises a groove (12dd) arranged at the bottom thereof, serving as a guide, sleeve, or enclosure for the second internal traction assembly (21), allowing movement of said second assembly (21) and forcing or limiting said assembly's (21) movement normal to the groove (12dd).

6. The exoskeleton system (1) according to any of the preceding claims, wherein the distal (10), middle (11) prosthetic phalanges, and coupling ring (12) are made of any material selected from the group consisting of polyvinyl chloride (PVC), low-density polyethylene (LDPE), polypropylene (PP), polystyrene (PS), flexible polyethylene, urethane, nylon, and/or any combination thereof, as well as metals such as steel, titanium, aluminum, alloys, mixtures thereof, and/or copolymers and/or combinations thereof.

7. The exoskeleton system (1) according to claim 1, wherein the traction mechanism is capable of modifying the state or configuration of the exoskeleton system (1) from a flexed to an extended configuration and vice versa based on the movement of the patient's metacarpophalangeal joint, wherein said traction mechanism is divided into:
an internal traction assembly, and
an external traction assembly.

8. The exoskeleton system (1) according to claim 7, wherein the internal traction assembly includes
a first internal traction assembly (20) in the form of a rod, arranged on both sides of the exoskeleton system (1), each end of which comprises a bore (20a) configured to concentrically match a bore (10ab) of the distal prosthetic phalanx (10) and on the other side, concentrically match a bore (12ab) of the coupling ring (12), and
a second internal traction assembly (21) in the form of a rod, arranged on both sides of the exoskeleton system (1), each end of which comprises a bore (21a) configured to concentrically match the bore (11cb) of the middle prosthetic phalanx (11) and on the other side, concentrically match a bore (40ab) of the movable ring (40).

9. The exoskeleton system (1) according to any of the preceding claims, wherein the first and second internal traction assemblies (20, 21) are manufactured with a length ranging from 2 cm to 10 cm and are made of any material selected from the group consisting of steel, aluminum, titanium, alloys, and/or combinations thereof.

10. The exoskeleton system (1) according to claim 7, wherein the external traction assembly includes
a movable ring (40) configured to move over the hollow elongated geometric body (12c) of the coupling ring (12), in response to the movement of the patient's metacarpophalangeal joint,
an extender or lever (50), capable of connecting to the movable ring (40) to transmit movement and displacement related to the movement of the patient's metacarpophalangeal joint, and
a multi-perforated connector (60), configured to secure and adapt to the dorsal part of the hand and/or wrist of the patient using attachment means and act as a stationary base for the extender or lever (50).

11. The exoskeleton system (1) according to claim 10, wherein the movable ring (40) is capable of moving up to a minimum stroke when said ring (40) contacts the protruding portion (12d) and, on the other hand, is capable of moving up to a maximum stroke when the ring (40) moves away from the protruding portion (12d), in the proximal direction towards the patient's hand, wherein furthermore, the movable ring (40) comprises
an upper coupling tab (40a) with a bore (40aa) configured to concentrically match bores (50a) of the extender or lever (50) to connect said ring (40) with the extender (50), and
a bore (40ab) at its bottom, configured to concentrically match the bore (21a) at the end of the second internal traction assembly (21) to connect said ring (40) with the second internal traction assembly (21).

12. The exoskeleton system (1) according to claim 10, wherein the extender or lever (40) has a curved design to increase component strength, mainly against forces resulting from the metacarpophalangeal joint movement, as well as efficiently transmit movement derived from it, and further comprises
a bore (50a) at each end configured to concentrically match, respectively, on one side, the bore (40aa) of the upper coupling tab (40a) of the movable ring (40), and on the other side, a bore (60a) of the multi-perforated connector (60), thereby connecting the movable ring (40) with the multi-perforated connector (60).

13. The exoskeleton system (1) according to claim 10, wherein the multi-perforated connector (60) comprises:
a set of bores (60a) distributed along the surface of said connector (60), configured to concentrically match the bore (50a) at the corresponding end of the extender or lever (50), thereby connecting said extender or lever (50) with the multi-perforated connector (60),
wherein the patient can place and attach the bore (50a) of the extender or lever (50) to any of the bores (60a) of the multi-perforated connector (60), to adapt the exoskeleton system (1) based on the patient's hand and/or wrist size, as well as the distance between the patient's remaining stump or proximal phalanx and the dorsal part and/or wrist of their hand, depending on the position where the multi-perforated connector (60) is placed.

14. The exoskeleton system (1) according to any of the preceding claims, wherein when any of the bores (10aa, 11aa, 11ba, 12aa) of the coupling tabs (10a, 11a, 11b, 12a) are coincidentally and concentrically placed, a fastening means is inserted into said bores to correspondingly and respectively connect the distal (10), middle (11) phalanges, and coupling ring (12) together,
wherein the fastening means is any selected from the group consisting of bolts, rivets, pins, threaded screw-nut systems, or combinations thereof.

15. The exoskeleton system (1) according to any of the preceding claims, wherein the external traction assembly (40, 50, 60) is made of any material selected from the group consisting of polyvinyl chloride (PVC), low-density polyethylene (LDPE), polypropylene (PP), polystyrene (PS), flexible polyethylene, urethane, nylon, and/or any combination thereof, as well as metals such as steel, titanium, aluminum, alloys, mixtures thereof, copolymers, and/or combinations thereof.

16. The exoskeleton system (1) according to claims 2 to 5, wherein the coupling ring (12) has an inner size ranging from 10 mm to 50 mm, and a total length (LTAC) ranging from 5 mm to 60 mm.

17. The exoskeleton system (1) according to claims 2 to 5 and 16, wherein the diameter of the protruding portion (12d) of the coupling ring (12) is larger than the diameter of the hollow elongated geometric body (12c), by 2 mm to 10 mm greater than the hollow elongated geometric body (12c).

18. The exoskeleton system (1) according to claims 2 to 5 and 16, wherein the movable ring (40) has an identical or slightly smaller diameter than the diameter of the protruding portion (12d) of the coupling ring (12).

19. The exoskeleton system (1) according to claims 2 to 5 and 16 to 18, wherein the diameter of the prosthetic phalanges (10, 11) has proportional, similar, or identical dimensions to the diameter of the coupling ring (12), so that these prosthetic phalanges (10, 11) are uniform and demonstrate continuity with the coupling ring (12), favoring the shape and aesthetics of the exoskeleton system (1).

20. The exoskeleton system (1) according to any of the preceding claims, wherein the internal traction assembly (20, 21) and the external traction assembly (40, 50, 60) are made of polyvinyl chloride (PVC), low-density polyethylene (LDPE), polypropylene (PP), polystyrene (PS), flexible polyethylene, urethane, steel, titanium, alloys, cast iron, iron, and/or combinations thereof.

21. The exoskeleton system (1) according to any of the preceding claims, wherein when any of the bores (10ab, 11cb, 12ab, 40ab) configured to receive the bores (20a, 21a) of the first and second internal traction assemblies (20, 21) are coincidentally and concentrically positioned, a fastening means is inserted into said bores to correspondingly and respectively join the internal traction assemblies (20, 21) with the respective components,
wherein the fastening means are any selected from the group consisting of bolts, rivets, pins, screw-nut systems, or combinations thereof.

22. The exoskeleton system (1) according to claim 10, wherein the length of the extender or lever (50) ranges from 5 cm to 30 cm, measured horizontally (in projection) from each bore (50a) placed at both ends of said extender (50).

23. The exoskeleton system (1) according to claim 10, wherein the multiperforated connector (60) may have a regular shape such as a rectangle, circle, square, polygon, or an irregular shape, such as a fan, half-circle, trapezoid, or combinations thereof, or in the shape of a glove, thereby favoring comfortable and ergonomic coupling on the dorsal part of the hand and/or wrist of the patient and may measure from 5 cm to 15 cm taking as reference the shape adopted by said connector (60) inscribed in a circle with said measurements.

24. The exoskeleton system (1) according to claim 10, wherein the multiperforated connector (60) comprises from 1 to n number of perforations where said perforations range from 2 mm to 10 mm, distributed radially, longitudinally, in a pattern established such as rectangular, circular, rectangular, polygonal, etc.

25. The exoskeleton system (1) according to any of the preceding claims, wherein
when the exoskeleton system (1) is in the extended configuration, the extender or lever (50) drives and pushes the movable ring (40), displacing it over the hollow elongated geometric body (12c) up to a minimum stroke (when the movable ring (40) comes into contact with the protruding portion (12d) of the coupling ring (12)), thus displacing both bars of the internal traction assemblies (20, 21) which function similarly to a parallel bar mechanism, forcing the prosthetic phalanges (10, 11) to align with the coupling ring (12), and;
when the exoskeleton system (1) transitions from the extended to the flexed configuration, the extender or lever (50) drives, pulls, or pushes the movable ring (40), displacing it over the hollow elongated geometric body (12c) up to a maximum stroke (when the movable ring (40) moves away from the protruding portion (12d) of the coupling ring (12), proximally towards the patient's hand), thus displacing both bars of the internal traction assemblies (20, 21) which function similarly to a parallel bar mechanism, causing the prosthetic phalanges (10, 11) to rotate, generating an angle from 0° to approximately 90°, from 0° to 95°, from 0° to 85°, measured from the middle prosthetic phalanx (11) relative to the coupling ring (12), with the coupling ring (12).

26. The exoskeleton system (1) according to any of the preceding claims, wherein when the patient moves their proximal phalanx to switch between any of the flexion or extended configurations, the prosthetic phalanges (10, 11) maintain their respective position in the corresponding configuration, even when there is an external force opposing or attempting to displace the prosthetic phalanges (10, 11) until the patient rotates their proximal phalanx again.
